(19) Europäisches Patentamt
European Patent Office
Office européen des brevets

(11) Veröffentlichungsnummer : **0 280 654 B1**

(12) **EUROPÄISCHE PATENTSCHRIFT**

(45) Veröffentlichungstag der Patentschrift : **13.05.92 Patentblatt 92/20**

(51) Int. Cl.$^5$ : **D06P 1/642,** D06P 3/54, C07D 239/42

(21) Anmeldenummer : **88810106.0**

(22) Anmeldetag : **22.02.88**

(54) **Verfahren zur Verbesserung der fotochemischen Stabilität von Färbungen auf Polyesterfasermaterialien.**

(30) Priorität : **27.02.87 CH 751/87**
**01.10.87 CH 3820/87**

(43) Veröffentlichungstag der Anmeldung : **31.08.88 Patentblatt 88/35**

(45) Bekanntmachung des Hinweises auf die Patenterteilung : **13.05.92 Patentblatt 92/20**

(84) Benannte Vertragsstaaten : **AT BE CH DE ES FR GB IT LI SE**

(56) Entgegenhaltungen :
**GB-A- 1 029 045**
**US-A- 3 660 404**
**CHEMICAL ABSTRACTS, Band 81, Nr. 4, Juli 1974, Seite 69, Zusammenfassung Nr. 14635v, Columbus, Ohio, US**

(73) Patentinhaber : **CIBA-GEIGY AG**
**Klybeckstrasse 141**
**CH-4002 Basel (CH)**

(72) Erfinder : **Reinert, Gerhard, Dr.**
**Weiherweg 1/7**
**CH-4123 Allschwil (CH)**
Erfinder : **Burdeska, Kurt, Dr.**
**Laufenburgerstrasse 30**
**CH-4058 Basle (CH)**

**EP 0 280 654 B1**

Anmerkung : Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die vorliegende Erfindung betrifft ein Verfahren zur Verbesserung der fotochemischen Stabilität von Färbungen auf Polyesterfasermaterialien.

Gefärbtes Polyesterfasermaterial wird bei Belichtung und besonders bei gleichzeitiger Wärmeeinwirkung geschädigt. Man hat daher solche gefärbten Materialien gegen die Licht- und Wärmeeinwirkung mit UV-Absorbern vom Benzophenon- oder Benzotriazoltyp geschützt ohne aber befriedigende Resultate zu erreichen, weil diese Verbindungen ihrer ungenügenden Sublimationsechtheit wegen, bei der Thermofixierung der Färbungen und bei Belichtung bei erhöhten Temperaturen zu Produkteverlust und somit ungenügendem Schutz geführt haben.

Die Stabilisierung von lichtempfindlichen organischen Materialien, wie z.B. vollsynthetischen Polymeren und natürlichen Polymeren vor allem reinen Additions- und reinen Kondensationspolymeren oder durch Additionspolymerisation vernetzten Kondensationspolymeren, z.B. Polyesterharzen ist aus der GB-A-1 029 045 bekannt. Es handelt sich aber dabei um den Schutz dieser organischen Materialien durch Einverleibung der Schutzmittel in die organische polymere Masse, wobei 2-o-Hydroxyphenyl-pyrimidine eingesetzt werden.

Die der vorliegenden Erfindung zugrundeliegende Aufgabe war es, ein Verfahren zur Verbesserung der fotochemischen Stabilität von Färbungen auf Polyesterfasermaterialien zu finden, welches keinen Produkteverlust aufweist und den gegenwärtigen Anforderungen genügt.

Diese Aufgabe wird durch die kennzeichnenden Merkmale von Anspruch 1 gelöst.

Gegenstand der vorliegenden Erfindung ist somit ein Verfahren zur Verbesserung der fotochemischen Stabilität von Färbungen auf Polyesterfasermaterialien mit UV-Absorbern, welches dadurch gekennzeichnet ist, dass man das Fasermaterial vor dem Färben oder wäbrend des Färbens mit einer Verbindung der Formel

(1) 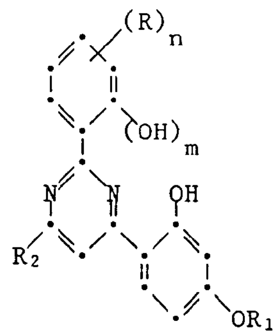

behandelt, worin
R $C_1$-$C_{18}$-Alkyl, $C_1$-$C_{18}$-Alkoxy, Halogen oder Hydroxy,
$R_1$ $C_1$-$C_{12}$-Alkyl
$R_2$ Wasserstoff, Halogen, $C_1$-$C_{18}$-Alkyl, $C_1$-$C_{12}$-Alkylamino, Hydroxy-$C_1$-$C_{12}$-alkylamino, Ar-$C_1$-$C_3$-alkylamino, Alkoxyalkylamino mit insgesamt 2-8 C-Atomen, $C_2$-$C_{18}$-Alkenyl, $C_1$-$C_{18}$-Alkoxy, durch Hydroxy, Carboxy oder $C_2$-$C_5$-Alkoxycarbonyl substituiertes $C_1$-$C_{18}$-Alkoxy, $C_2$-$C_{18}$-Alkenyloxy, Phenyl oder durch Halogen, $C_1$-$C_4$-Alkyl, Hydroxy-$C_1$-$C_4$-alkyl, $C_2$-$C_{18}$-Alkenyloxy, $C_1$-$C_{18}$Alkoxy, Hydroxy, Hydroxy-$C_1$-$C_4$-alkoxy oder Carboxy-$C_1$-$C_4$-alkoxy substituiertes Phenyl,
m 0 oder 1 und
n 0, 1 oder 2
bedeuten.

Alkyl- und Alkoxyreste, R und $R_2$ mit 1 bis 18 C-Atomen sind Reste wie Methyl, Ethyl, Propyl, Isopropyl, n-Butyl, tert.-Butyl, Pentyl, Hexyl, Octyl, Decyl, Dodecyl, Tetradecyl, Hexadecyl, Octadecyl (Stearyl), Methoxy, Ethoxy, Propoxy, Isopropoxy, n-Butoxy, tert.-Butoxy, Pentyloxy, Hexyloxy, Octyloxy, Decyloxy, Dodecyloxy und Octadecyloxy. Alkylreste $R_1$ weisen 1 bis 12, vorzugsweise 1 bis 8 C-Atome auf. Sie sind Reste wie Methyl, Ethyl, Propyl, Isopropyl, n-Butyl, tert.-Butyl, Pentyl, Hexyl, Octyl, Decyl und Dodecyl.

$C_2$-$C_{18}$-Alkenylreste $R_2$ sind Reste, wie Vinyl, Allyl, Butenyl, Isobutenyl, Pentenyl, Hexenyl, Heptenyl, Octenyl, Decenyl, Dodecenyl, Tetradecenyl und Octadecenyl.

Als $C_2$-$C_{18}$-Alkenyloxyreste $R_2$ kommen Reste, wie Vinyloxy, Allyloxy, Butenyloxy, Isobutenyloxy, Pentenyloxy, Hexenyloxy, Heptenyloxy, Octenyloxy, Decenyloxy, Dodecenyloxy, Tetradecenyloxy und Octadecenyloxy in Betracht.

Alkyl- und Hydroxyalkylaminoreste $R_2$ mit im Alkylteil 1 bis 12 und vorzugsweise 2 bis 8 C-Atomen sind

Reste, wie der Methyl-, Ethyl-, Propyl-, Isopropyl-, n-Butyl, tert.-Butyl, Pentyl-, Hexyl-, Octyl-, Decyl- und Dodecylaminorest, sowie die gleichen durch eine vorzugsweise endständige Hydroxygruppe substituierten Reste.

Alkoxyalkylaminoreste $R_2$ mit insgesamt 2 bis 8 C-Atomen sind Reste wie Methoxymethyl-, Methoxyethyl-, Methoxypropyl-, Methoxybutyl-, Ethoxyethyl-, Ethoxypropyl-, Ethoxybutyl-, Ethoxypentyl-, Propoxyethyl-, Propoxypropyl-, Butoxyethyl-, Butoxypropyl- und Butoxybutylamino.

Aralkylaminoreste $R_2$ sind vorzugsweise Phenylalkylreste mit 1 bis 3 C-Atomen im Alkylteil wie Benzyl-, Phenethyl- und Phenylpropylamino.

Als Halogensubstituente R und $R_2$ kommen Brom- und vorzugsweise Chloratome in Betracht.

m und n sind vorzugsweise 0.

Bevorzugte Verbindungen der Formel (1) sind solche worin $R_2$ Wasserstoff, Halogen, $C_1$-$C_{18}$-Alkyl, $C_2$-$C_{18}$-Alkenyl, $C_1$-$C_{18}$-Alkoxy, durch Hydroxy, Carboxy oder $C_2$-$C_5$-Alkoxycarbonyl substituiertes $C_1$-$C_{18}$-Alkoxy, $C_2$-$C_{18}$-Alkenyloxy, Phenyl oder durch Halogen, $C_1$-$C_4$-Alkyl, Hydroxy-$C_1$-$C_4$-alkyl, $C_2$-$C_{18}$-Alkenyloxy, $C_1$-$C_{18}$-Alkoxy, Hydroxy oder Carboxy-$C_1$-$C_4$-alkoxy substituiertes Phenyl bedeutet.

Von besonderem Interesse für das erfindungsgemässe Verfahren sind die Verbindungen der Formel

(2) 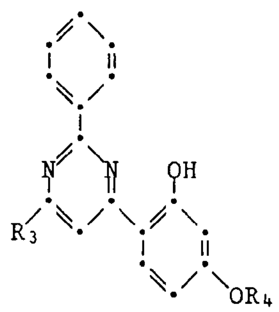

worin

$R_3$ Wasserstoff, Chlor, $C_1$-$C_4$-Alkyl, $C_1$-$C_8$-Alkylamino, Hydroxy-$C_1$-$C_4$-alkylamino, Benzylamino, Phenethylamino, $C_1$-$C_3$-Alkoxy-$C_2$-$C_4$-alkylamino, Phenyl oder durch $C_1$-$C_4$-Alkyl, Hydroxy, $C_1$-$C_{18}$- Alkoxy, $C_2$-$C_{18}$-Alkenyloxy, Hydroxy-$C_1$-$C_4$-alkoxy oder Carboxy-$C_1$-$C_4$-alkoxy substituiertes Phenyl und

$R_4$ $C_1$-$C_8$-Alkyl

bedeuten.

Bevorzugte Verbindungen der Formel (2) sind jene worin $R_3$ Wasserstoff, Chlor, $C_1$-$C_4$-Alkyl, Phenyl oder durch $C_1$-$C_4$-Alkyl, Hydroxy, $C_1$-$C_{18}$-Alkoxy, $C_2$-$C_{18}$-Alkenyloxy, Hydroxy-$C_1$-$C_4$-alkoxy oder Carboxy-$C_1$-$C_4$-alkoxy substituiertes Phenyl bedeutet.

Im erfindungsgemässen Verfahren werden bevorzugt Verbindungen der Formel

(3) 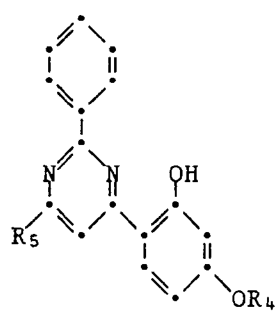

verwendet, worin

$R_4$ die oben angegebene Bedeutung hat und

$R_5$ $C_1$-$C_4$-Alkyl, Chlor, Phenyl, o-Hydroxyphenyl, $C_1$-$C_8$-Alkylamino, Hydroxy-$C_1$-$C_4$-alkylamino, Phenethylamino oder $C_1$-$C_3$-Alkoxy-$C_2$-$C_3$-alkylamino

bedeuten.

In den Verbindungen der Formel (3) ist $R_5$ vorzugsweise $C_1$-$C_4$-Alkyl, Chlor, Phenyl oder o-Hydroxyphenyl.

Die Verbindungen der Formeln (1) bis (3), die auch als UV-Absorber bekannt geworden sind, sind zum Teil bekannt und können in an sich bekannter Weise hergestellt werden, so z.B. durch Umsetzen, in Gegenwart von Friedel-Crafts-Katalysatoren, von Halogenpyrimidinen und Verbindungen der Benzolreihe, die eine gegebenenfalls veretherte Hydroxylgruppe in Nachbarstellung zur entstehenden Bindung an den Pyrimidinring aufweisen und gegebenenfalls andere Verbindungen der Benzolreihe (vgl. z.B. Britische Patentschrift 1 029 045).

Die neuen erfindungsgemäss verwendbaren Verbindungen entsprechen der Formel

(4)

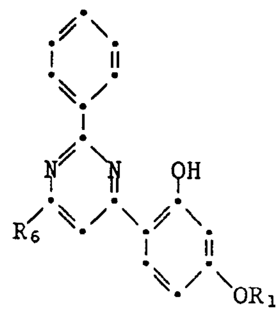

worin

$R_1$ die oben angegebene Bedeutung hat und

$R_6$ $C_1$-$C_8$-Alkylamino, Hydroxy-$C_1$-$C_4$-alkylamino, Phenethylamino oder $C_1$-$C_3$-Alkoxy-$C_2$-$C_3$-alkylamino bedeuten. Sie können dadurch hergestellt werden, dass man ein Mol 4,6-Dichlor-2-phenylpyrimidin mit einem Mol Resorcin in Gegenwart eines Friedel-Crafts-Katalysators umsetzt und das erhaltene monochlorierte Produkt nach an sich bekannten Methoden alkyliert.

Geeignete bekannte Verbindungen der Formeln (1), (2) und (3) sind z.B.

2,4-Diphenyl-6-(2′-hydroxy-4′-methoxyphenyl)-pyrimidin, F. 174-175°C;
2,4-Diphenyl-6-(2′-hydroxy-4′-ethoxyphenyl)-pyrimidin, F. 177-178°C;
2,4-Diphenyl-6-(2′-hydroxy-4′-isopropoxyphenyl)-pyrimidin, F. 123-124°C;
2,4-Diphenyl-6-(2′-hydroxy-4′-butoxyphenyl)-pyrimidin, F. 148-149°C;
2,4-Diphenyl-6-(2′-hydroxy-4′-heptyloxyphenyl)-pyrimidin, F. 103-104°C;
2-Phenyl-4-chlor-6-(2′-hydroxy-4′-ethoxyphenyl)-pyrimidin, F. 155-156°C;
2-Phenyl-4-methyl-6-(2′-hydroxy-4′-ethoxyphenyl)-pyrimidin, F. 125-126°C;
2-Phenyl-4,6-di-(2′-hydroxy-4′-ethoxyphenyl)-pyrimidin, F. 250°C.

Die als UV-Absorber zu verwendenden Verbindungen der Formeln (1), (2) und (3) werden in einer Menge von 0,05 bis 7,5, vorzugsweise 0.20 bis 3 und besonders 0,5 bis 2 % vom Gewicht des Fasermaterials eingesetzt.

Als Polyesterfasermaterial, das in Gegenwart der genannten UV-Absorber gefärbt werden kann, sind z.B. Celluloseesterfasern, wie Cellulose-2 $\frac{1}{2}$-acetatfasern und -triacetatfasern und besonders lineare Polyesterfasern zu erwähnen. Unter linearen Polyesterfasern sind dabei Synthesefasern zu verstehen, die z.B. durch Kondensation von Terephthalsäure mit Ethylenglykol oder von Isophthalsäure oder Terephthalsäure mit 1,4-Bis(hydroxymethyl)-cyclohexan erhalten werden, sowie Mischpolymere aus Terephthal- und Isophthalsäure und Ethylenglykol. Der in der Textilindustrie bisher fast ausschliesslich eingesetzte lineare Polyester besteht aus Terephthalsäure und Ethylenglykol.

Die Fasermaterialien können auch als Mischgewebe unter sich oder mit anderen Fasern, z.B. Mischungen aus Polyacrylnitril/Polyester, Polyamid/Polyester, Polyester/Baumwolle, Polyester/Viskose und Polyester/Wolle, verwendet werden.

Das zu färbende Textilmaterial kann sich in verschiedenen Aufmachungsformen befinden. Vorzugsweise kommt Stückware, wie Gewirke oder Gewebe in Betracht.

Die zu verwendenden Dispersionsfarbstoffe, die in Wasser nur sehr wenig löslich sind und in der Farbflotte zum grössten Teil in Form einer feinen Dispersion vorliegen, können den verschiedensten Farbstoffklassen angehören, beispielsweise den Acridon-, Azo-, Anthrachinon-, Cumarin-, Methin-, Perinon-, Naphthochinonimin-, Chinophthalon-, Styryl- oder Nitrofarbstoffen. Es können auch Mischungen von Dispersionsfarbstoffen erfindungsgemäss eingesetzt werden.

Polyester/Wolle-Mischfasermaterialien werden erfindungsgemäss vorzugsweise mit handelsüblichen Mischungen von anionischen Farbstoffen und Dispersionsfarbstoffen gefärbt. Bei den anionischen Farbstoffen handelt es sich beispielsweise um Salze schwermetallhaltiger oder vorzugsweise metallfreier Mono-, Dis- oder Polyazofarbstoffe einschliesslich der Formazanfarbstoffe sowie der Anthrachinon-, Xanthen-, Nitro-, Triphenylmethan-, Naphthochinonimin- und Phthalocyaninfarbstoffe. Der anionische Charakter dieser Farbstoffe kann durch Metallkomplexbildung allein und/oder vorzugsweise durch saure, salzbildende Substituenten, wie Carbonsäuregruppen, Schwefelsäure- und Phosphonsäureestergruppen, Phosphonsäuregruppen oder Sulfonsäuregruppen bedingt sein. Diese Farbstoffe können im Molekül auch sogenannte reaktive Gruppierungen,

welche mit der Wolle eine kovalente Bindung eingehen, aufweisen.

Von Interesse sind insbesondere die 1:1- oder 1:2-Metallkomplexfarbstoffe. Die 1:1-Metallkomplexfarbstoffe weisen vorzugsweise eine oder zwei Sulfonsäuregruppen auf. Als Metall enthalten sie ein Schwermetallatom wie z.B. Kupfer, Nickel oder insbesondere Chrom.

Die 1:2-Metallkomplexfarbstoffe enthalten als Zentralatom ein Schwermetallatom wie z.B. ein Kobaltatom oder insbesondere ein Chromatom. Mit dem Zentralatom sind zwei komplexbildende Komponenten verbunden, von denen mindestens eine ein Farbstoffmolekül ist, vorzugsweise jedoch beide Farbstoffmoleküle sind. Dabei können die beiden an der Komplexbildung beteiligten Farbstoffmoleküle gleich oder voneinander verschieden sein. Die 1:2-Metallkomplexfarbstoffe können z.B. zwei Azomethinmoleküle, einen Disazofarbstoff und einen Monoazofarbstoff oder vorzugsweise zwei Monoazofarbstoffmoleküle enthalten. Die Azofarbstoffmoleküle können wasserlöslichmachende Gruppen aufweisen, wie z.B. Säureamid-, Alkylsulfonyl- oder die obengenannten sauren Gruppen. Bevorzugt sind 1:2-Kobalt- oder 1:2-Chromkomplexe von Monoazofarbstoffen, die Säureamid-, Alkylsulfonyl- oder insgesamt eine einzige Sulfonsäuregruppe aufweisen.

Es können auch Mischungen der anionischen Farbstoffe eingesetzt werden.

Fasermischungen aus Polyester und Baumwolle werden in der Regel mit Kombination von Dispersionsfarbstoffen und Küpenfarbstoffen, Schwefelfarbstoffen, Leukoküpenesterfarbstoffen, Direktfarbstoffen oder Reaktivfarbstoffen gefärbt, wobei der Polyesteranteil mit Dispersionsfarbstoffen vor-, gleichzeitig oder nachgefärbt wird.

Bei den Küpenfarbstoffen handelt es sich um höher annellierte und heterocyclische Benzochinone oder Naphthochinone, um Schwefelfarbstoffe und insbesondere um anthrachinoide oder indigoide Farbstoffe. Beispiele von erfindungsgemäss verwendbaren Küpenfarbstoffen sind im Colour Index, 3rd Edition (1971), Vol. 3, auf den Seiten 3649 bis 3837 unter der Bezeichnung "Sulphur Dyes" und "Vat Dyes" aufgeführt.

Als Direktfarbstoffe geeignet sind beispielsweise die in Colour Index 3rd Edition (1971), Vol. 2, auf den Seiten 2005 bis 2478 genannten "Direct Dyes".

Die Leukoküpenesterfarbstoffe sind z.B. aus Küpenfarbstoffen der Indigo-, Anthrachinon- oder Indanthren-Reihe durch Reduktion z.B. mit Eisenpulver und anschliessende Veresterung z.B. mit Chlorsulfonsäure erhältlich und sind im Colour Index, 3rd Edition (1971), Vol. 3, als "Solubilised Vat Dyes" bezeichnet.

Unter Reaktivfarbstoffen werden die üblichen Farbstoffe verstanden, welche mit der Cellulose eine chemische Bindung eingehen, z.B. die im Colour Index, 3rd Edition (1971), Vol. 3, auf den Seiten 3391 bis 3560 aufgeführten "Reactive Dyes".

Die Menge der der Flotte zuzusetzenden Farbstoffe richtet sich nach der gewünschten Farbstärke; im allgemeinen haben sich Mengen von 0,01 bis 10, vorzugsweise 0,02 bis 5 Gewichtsprozent, bezogen auf das eingesetzte Textilmaterial, bewährt.

Die erfindungsgemäss zu verwendenden Verbindungen können auch in Mischung mit bekannten Carrier auf Basis von z.B. Di- oder Trichlorbenzol, Methyl- oder Ethylbenzol, o-Phenylphenol, Benzylphenol, Diphenylether, Chlordiphenyl, Methyldiphenyl, Cyclohexanon, Acetophenon, Alkylphenoxyethanol, Mono-, Di- oder Trichlorphenoxyethanol oder -propanol, Pentachlorphenoxyethanol, Alkylphenylbenzoate, oder insbesondere auf Basis von Diphenyl, Methyldiphenylether, Dibenzylether, Methylbenzoat, Butylbenzoat und Phenylbenzoat eingesetzt werden.

Die Carrier werden vorzugsweise in einer Menge von 0,5 bis 2 g/ℓ Flotte oder 5 bis 10 Gewichtsprozent, bezogen auf die zu verwendenden Verbindungen eingesetzt.

Die Farbbäder können je nach dem zu behandelnden Textilmaterial neben den Farbstoffen und den erfindungsgemäss einzusetzenden Verbindungen Wollschutzmittel, Oligomereninhibitoren, Oxidationsmittel, Antischaummittel, Emulgatoren, Egalisiermittel, Retarder und vorzugsweise Dispergiermittel enthalten.

Die Dispergiermittel dienen vor allem zur Erzielung einer guten Feinverteilung der Dispersionsfarbstoffe. Es kommen beim Färben mit Dispersionsfarbstoffen die allgemein gebräuchlichen Dispergatoren in Frage.

Als Dispergiermittel kommen vorzugsweise sulfatierte oder phosphatierte Anlagerungsprodukte von 15 bis 100 Mol Ethylenoxid oder vorzugsweise Propylenoxid an mehrwertige, 2 bis 6 Kohlenstoffatome aufweisende aliphatische Alkohole wie z.B. Ethylenglykol, Glycerin oder Pentaerythrit oder an mindestens zwei Aminogruppen oder eine Aminogruppe und eine Hydroxylgruppe aufweisende Amine mit 2 bis 9 Kohlenstoffatomen sowie Alkylsulfonate mit 10 bis 20 Kohlenstoffatomen in der Alkylkette, Alkylbenzolsulfonate mit geradkettiger oder verzweigter Alkylkette mit 8 bis 20 Kohlenstoffatomen in der Alkylkette, wie z.B. Nonyl-oder Dodecylbenzolsulfonat, 1,3,5,7-Tetramethyloctylbenzolsulfonat oder Octadecylbenzolsulfonat, sowie Alkylnaphthalinsulfonate oder Sulfobernsteinsäureester, wie Natriumdioctylsulfosuccinat, in Betracht.

Besonders günstig haben sich als anionische Dispergiermittel Ligninsulfonate, Polyphosphate und vorzugsweise Formaldehyd-Kondensationsprodukte aus aromatischen Sulfonsäuren, Formaldehyd und gegebenenfalls mono- oder bifunktionellen Phenolen wie z.B. aus Kresol, β-Naphtholsulfonsäure und Formaldehyd, aus Benzolsulfonsäure, Formaldehyd und Naphthalinsäure, aus Naphthalinsulfonsäure und Formaldehyd oder

aus Naphthalinsulfonsäure, Dihydroxydiphenylsulfon und Formaldehyd erwiesen. Bevorzugt ist das Dinatriumsalz des Di-(6-sulfonaphthyl-2-)methans.

Es können auch Gemische von anionischen Dispergiermitteln zum Einsatz kommen. Normalerweise liegen die anionischen Dispergiermittel in Form ihrer Alkalimetallsalze, Ammoniumsalze oder Aminsalze vor. Diese Dispergiermittel werden vorzugsweise in einer Menge von 0,1 bis 5 g/ℓ Flotte verwendet.

Die Färbebäder können je nach dem zu verwendenden Farbstoff und Substrat zusätzlich zu den bereits genannten Hilfsmitteln auch übliche Zusätze, zweckmässig Elektrolyte wie Salze, z.B. Natriumsulfat, Ammoniumsulfat, Natrium- oder Ammoniumphosphate oder -polyphosphate, Metallchloride oder -nitrate wie Natriumchlorid, Calciumchlorid, Magnesiumchlorid bzw. Calciumnitrate, Ammoniumacetat oder Natriumacetat und/oder Säuren, z.B. Mineralsäuren wie Schwefelsäure oder Phosphorsäure, oder organische Säuren, zweckmässig nieder aliphatische Carbonsäuren wie Ameisen-, Essig- oder Oxalsäure sowie auch Alkalien, Alkalispender oder Komplexbildner, enthalten. Die Säuren dienen vor allem der Einstellung des pH-Wertes der erfindungsgemäss verwendeten Flotten, der in der Regel 4 bis 6,5, vorzugsweise 4,5 bis 6, beträgt.

Die Färbungen erfolgen mit Vorteil aus wässeriger Flotte nach dem Ausziehverfahren. Das Flottenverhältnis kann dementsprechend innerhalb eines weiten Bereichs gewählt werden, z.B. 1:4 bis 1:100, vorzugsweise 1:6 bis 1:50. Die Temperatur, bei der gefärbt wird, beträgt mindestens 50°C und in der Regel ist sie nicht höher als 140°C. Vorzugsweise liegt sie im Bereich von 80 bis 135°C.

Lineare Polyesterfasern und Cellulosetriacetatfasern färbt man vorzugsweise nach dem sogenannten Hochtemperaturverfahren in geschlossenen und zweckmässigerweise auch druckbeständigen Apparaturen bei Temperaturen von über 100°C, bevorzugt zwischen 110 und 135°C, und unter Druck. Als geschlossene Gefässe eignen sich beispielsweise Zirkulationsapparaturen wie Kreuzspul-oder Baumfärbeapparate, Haspelkufen, Düsen- oder Trommelfärbemaschinen, Muff-Färbeapparate, Paddeln oder Jigger.

Cellulose-2½-acetatfasern färbt man vorzugsweise bei Temperaturen von 80-85°C.

Das erfindungsgemässe Färbeverfahren kann so durchgeführt werden, dass man das Färbegut entweder zuerst mit den Verbindungen kurz behandelt und anschliessend färbt oder vorzugsweise gleichzeitig mit den Verbindungen und dem Farbstoff behandelt.

Vorzugsweise lässt man das Färbegut während 5 Minuten bei 50 bis 80°C im Bad, das den Farbstoff, die Verbindung und gegebenenfalls weitere Zusätze enthält und auf einen pH-Wert von 4,5 bis 5,5 eingestellt ist vorlaufen, steigert die Temperatur innerhalb von 10 bis 20 Minuten auf 100 bis 110°C und innerhalb von weiteren 10 bis 20 Minuten auf 125 bis 130°C, und belässt die Färbeflotte 15 bis 90 Minuten, vorzugsweise 30 Minuten, bei dieser Temperatur.

Die Fertigstellung der Färbungen erfolgt durch Abkühlen der Färbeflotte auf 50 bis 80°C, Spülen der Färbungen mit Wasser und gegebenenfalls durch Reinigung auf übliche Weise im alkalischen Medium unter reduktiven Bedingungen. Die Färbungen werden dann wiederum gespült und getrocknet. Bei einer allfälligen Verwendung von Carriern werden die Färbungen zwecks Verbesserung der Lichtechtheit, vorteilhafterweise nach einer Hitzebehandlung, z.B. Thermosolieren, unterworfen, die vorzugsweise bei 160 bis 180°C und während 30 bis 90 Sekunden durchgeführt wird. Bei Verwendung von Küpenfarbstoffen für den Celluloseanteil wird die Ware auf übliche Weise zuerst mit Hydrosulfit bei einem pH-Wert von 6 bis 12,5 und dann mit Oxydationsmittel behandelt und schliesslich ausgewaschen.

Mit dem erfindungsgemässen Verfahren werden Polyesterfasermaterialien fotochemisch stabilisiert, d.h. gegen Belichtung, insbesondere Heissbelichtung, mit sichtbarem und UV-Licht geschützt.

Ein besonders hervorragender Vorteil des erfindungsgemässen Verfahrens ist, dass im Vergleich zu bisher bekannten Verfahren zur fotochemischen Stabilisierung von Polyesterfasermaterialien keine Vor- oder Nachbehandlung des Fasermaterials erforderlich ist.

In den folgenden Herstellungsvorschriften und Beispielen beziehen sich die Prozentsätze, wenn nichts anderes angegeben ist, auf das Gewicht. Die Mengen beziehen sich bei den Farbstoffen und bei den UV-Absorbern auf Formulierungen. Allfällige fünfstellige Colour-Index-Nummern (C.I.) beziehen sich auf die 3. Auflage des Colour-Index.

## Herstellungsvorschrift A

### Herstellung einer Verbindung der Formel (1)

22,5 g 4,6-Dichlor-2-phenylpyrimidin und 11,0 g Resorcin werden in 150 ml Nitrobenzol gelöst und mit 13,3 g wasserfreiem Aluminiumchlorid versetzt. Anschliessend wird auf 75-80°C erhitzt und 12 Stunden lang bei dieser Temperatur gerührt. Nach dem Abkühlen auf Raumtemperatur und Zersetzung des Reaktionsgemisches mit Salzsäure und Wasser wird das Nitrobenzol mit Wasserdampf abdestilliert. Man erhält das 2-Phenyl-4-chlor-6-(2′,4′-dihydroxyphenyl)-pyrimidin in guter Ausbeute vom Smp. 213-214°C. Das Produkt wird dann nach

an sich bekannter Weise, z.B. gemäss Beispiel 9 der GB-A-1 029 045 mit Diethylsulfat alkyliert. Man erhält das 2-Phenyl-4-chlor-6-(2′-hydroxy-4′-ethoxyphenyl)-pyrimidin vom Smp. 155-156°C.

22,9 g 2-Phenyl-4-chlor-6-(2′-hydroxy-4′-ethoxyphenyl)-pyrimidin werden mit 12,8 g Ethanolamin und 120 ml Cyclohexanol während 3 Stunden auf 140-145°C erhitzt. Nach dem Abkühlen auf 100°C lässt man 120 ml Wasser in das Reaktionsgemisch einfliessen. Danach rührt man während 10 Minuten durch. Nach dem Abstellen des Rührers erfolgt eine Schichttrennung. Die wässrige Schicht wird von der Cyclohexanolschicht abgetrennt und letztere zur Trockene eingedampft. Das zurückbleibende Produkt wird aus Ethanol umkristallisiert und man erhält 22 g des fast farblosen Produktes der Formel

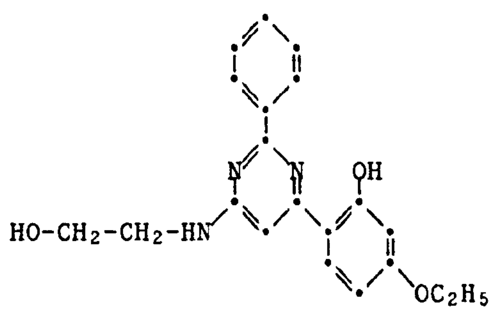

vom Smp. 162-164°C.

In ähnlicher Weise erhält man folgende Verbindungen:

2-Phenyl-4-(3′-hydroxypropylamino)-6-(2″-hydroxy-4″-ethoxyphenyl)-pyrimidin, Smp. 129-131°C, 2-Phenyl-4-(4′-hydroxybutylamino)-6-(2″-hydroxy-4″-ethoxyphenyl)-pyrimidin, Smp. 94-95°C; 2-Phenyl-4-ethylamino-6-(2′-hydroxy-4′-ethoxyphenyl)-pyrimidin, Smp. 107-108°C; 2-Phenyl-4-octylamino-6-(2′-hydroxy-4′-ethoxyphenyl)-pyrimidin, Oel; 2-Phenyl-4-methoxypropylamino-6-(2′-hydroxy-4′-ethoxyphenyl)-pyrimidin, Oel und 2-Phenyl-4-phenethylamino-6-(2′-hydroxy-4′-ethoxyphenyl)-pyrimidin, Smp. 117-118°C.

Herstellungsvorschrift B

Herstellung einer UV-Absorber-Dispersion

5 g eines UV-Absorbers werden mit 5 g des Kondensationsproduktes aus Naphthalinsulfonsäure und Formaldehyd als Dispergator, das in 7,5 ml Wasser gelöst ist, mit 20 g Quarzkügelchen (Durchmesser ca. 1 mm) versetzt und mit einem Rührer mit ca. 1600 Umdrehungen pro Minute so lange gemahlen, bis die Teilchengrösse unter 2 µm liegt. Die Dispersion wird von den Quarzkügelchen mittels eines feinen Maschensiebes abgetrennt und mit Wasser auf 20 % an Wirksubstanz eingestellt. Danach rührt man 0,3 % Carboxymethylcellulose ein, um die Dispersion zu stabilisieren.

Beispiel 1:

Es werden 13 Serien von Trikotstücken aus Diolen® von jeweils 3 Stücken von je 10 g in einem HT-Färbeapparat mit Flotten behandelt, die pro Liter

2 g Ammoniumsulfat

0,5 g eines Dispersionsstabilisators

0,25 g eines Dispergators

0,2 % C.I. Dispers Orange 53 (als ca. 35 % Dispersion) und je 1,65 oder 5,0 % der UV-Absorber (stets als 20%ige Dispersionen)

I 2-(2′-Hydroxy-3′-tert.-butyl-5′-methylphenyl)-5-chlorobenztriazol

II 2,2′-Dihydroxy-4,4′-dimethoxybenzophenon;

III 2-Phenyl-4-methyl-6-(2′-hydroxy-4′-ethoxyphenyl)-pyrimidin;

IV 2,4-Diphenyl-6-(2′-hydroxy-4′-ethoxyphenyl)-pyrimidin

V 2-Phenyl-4-chlor-6-(2′-hydroxy-4′-ethoxyphenyl)-pyrimidin oder

VI 2-Phenyl-4,6-di-(2′-hydroxy-4′-ethoxyphenyl)-pyrimidin

enthalten und die mit Ameisensäure auf pH 5 eingestellt werden. Ferner wird eine Färbung ohne UV-Absorber-Zusatz angefertigt.

Das Färben wird in Druckbomben bei einem Flottenverhältnis von 1:10 vorgenommen, indem man

zunächst die Trikotstücke bei 50°C während 5 Minuten behandelt, sodann die Temperatur zuerst in 10 Minuten auf 100°C und dann in weiteren 10 Minuten auf 130°C erhöht. Es wird 30 Minuten lang bei dieser Temperatur gefärbt, danach auf 50°C abgekühlt, mit warmem Wasser gespült, zentrifugiert und in einem Umluftofen bei 80°C getrocknet. Die 13 Serien von je 3 Stücken von je 10 g werden nun in 3 Serien unterteilt. Während die Serie 1 unbehandelt bleibt, werden die Serie 2, 60 Sekunden lang bei 180°C und die Serie 3, 60 Sekunden lang bei 200°C in einem Heissluft-Thermofixiergerät (z.B. das von W. Mathis, Niederhasli, Schweiz) behandelt.

Alle 39 Muster werden anschliessend auf ihre Heisslichtechtheit nach Ford EU BO 50-2 (Prüfvorschrift FLT EU BO 50-2; Prüfgerät Xenotest 1200, Gleichlauf; Prüfdauer 48 Stunden; Schwarztafeltemperatur 75°C; Feuchtigkeit 80 %) geprüft. Man erhält folgende Resultate:

Tabelle I:

| UV-Absorber | Menge | Lichtechtheiten nach FORD | | |
|---|---|---|---|---|
| | % | - | 180°C 60" | 200°C 60" |
| keiner | - | 1 - 2 | 1 - 2 | 1 - 2 |
| I | 1,65 | 3 - 4 | 3 - 4 | 2 - 3 |
| | 5,0 | - 4 | 3 - 4 | 3 |
| II | 1,65 | 3 - 4 | 3 - 4 | 3 |
| | 5,0 | 4 | 3 - 4 | 3 + |
| III | 1,65 | 3 - 4 | 3 - 4 | 3 - 4 |
| | 5,0 | 4 | 4 | 4 |
| IV | 1,65 | 3 - 4 | 3 - 4 | 3 - 4 |
| | 5,0 | 4 | 4 | 4 |
| V | 1,65 | 3 - 4 H | - 4 | 4 |
| | 5,0 | - 4 | 4 | - 4 - 5 |
| VI | 1,65 | 3 H | 3 H+ | 3 - 4 H |
| | 5,0 | 4 | 4 | 4 + |

Die Lichtechtheitsbewertung zeigt deutlich, dass bei den bekannten UV-Absorbern I und II eine deutliche Verringerung der Werte bei 200°C erfolgt, während bei den erfindungsgemäss verwendbaren UV-Absorbern die Werte gleich bleiben.

Beispiel 2:

Man geht wie in Beispiel 1 beschrieben vor, mit der Ausnahme, dass kein Farbstoff eingesetzt wird. Die Thermofixierungen werden unter denselben Bedingungen vorgenommen. Die auf der Faser befindlichen UV-Absorbermengen werden durch Remissionsmessungen auf den Trikotstücken bestimmt; als charakteristische Konzentrationsgrösse werden die K/S-Werte (Konzentration des UV-Absorbers auf dem Substrat in % der Anfangskonzentration = 100 %) angegeben.

Tabelle II:

| UV-Absorber | Menge % | * K/S - WERTE (in %) - | 180°C 60" | 200°C 60" |
|---|---|---|---|---|
| I | 1,65 | 25,1 (100 %) | 19,0 (75 %) | 11,3 (45 %) |
| | 5,0 | 57,1 (100 %) | 43,2 (75,7 %) | 28,7 (50,3 %) |
| II | 1,65 | 26,1 (100 %) | 21,5 (82,4 %) | 18,0 (68,9 %) |
| | 5,0 | 46,6 (100 %) | 31,9 (68,5 %) | 25,0 (53,6 %) |
| III | 1,65 | 23,7 (100 %) | 22,2 (93,7 %) | 17,8 (75,1 %) |
| | 5,0 | 49,0 (100 %) | 36,5 (74,5 %) | 29,9 (61,0 %) |
| IV | 1,65 | 22,3 (100 %) | 21,2 (95,5 %) | 16,2 (72,6 %) |
| | 5,0 | 48,5 (100 %) | 42,7 (88,0 %) | 43,9 (90,5 %) |
| V | 1,65 | 29,1 (100 %) | 29,2 (100 %) | 27,2 (93,5 %) |
| | 5,0 | 37,1 (100 %) | 41,2 (>100 %) | 50,5 (>100 %) |
| VI | 1,65 | 25,1 (100 %) | 22,6 (90 %) | 21,8 (87,0 %) |
| | 5,0 | 62,6 (100 %) | 67,4 (>100 %) | 68,4 (>100 %) |

* bei 340 nm gemessen

Die K/S-Werte ergeben eindeutig, dass der Verlust an UV-Absorber beim Thermofixieren für die Produkte I und II höher als für die Produkte III und IV ist. Die Produkte V und VI zeigen in den höheren Konzentrationen eine Tendenz zur Nachentwicklung.

Beispiel 3:

Auf 7 Strang von je 10 g eines Terylene®-Stapelgarns werden Graubraun-Färbungen mit und ohne UV-Absorber hergestellt, wobei man wie in Beispiel 1 beschrieben, mit der folgenden Farbstoffmischung färbt:

1,00 % C.I. Disperse Yellow 42
0.30 % C.I. Disperse Blue 60
0,15 % C.I. Disperse Violet 57
0.40 % C.I. Disperse Red 302

Als UV-Absorber verwendet man die Verbindungen I, III und V in Mengen von 1.5 % und 4,5 % (stets als 20 %-ige Dispersionen). Von den Färbungen werden nun die Heisslichtechtheiten nach Ford EU BO 50-2 (Belichtung 48 h und 96 h) und DIN 75.202, Entwurf (=Fakra Belichtung: 96 h und 192 h) bestimmt. Man erhält folgende Resultate:

Tabelle III:

| UV-Absorber | Menge | HEISSLICHTECHTHEITEN nach | | | |
|---|---|---|---|---|---|
| | % | Fakra 96h | Fakra 192h | Ford 48h | Ford 96h |
| keiner | - | 3 - 4 | 3 | 3 - 4 | 3 |
| I | 1,5 | 3 - 4 | 3 - 4 | 3 - 4 | 3 |
| | 4,5 | 4 - 5 | 4 | + 4 | 3 - 4 |
| III | 1,5 | 4 | 4 | 4 | 3 - 4 + |
| | 4,5 | -4 - 5 | - 4 | 4 + | 4 |
| V | 1,5 | 4 | 3 - 4 + | 3 - 4H+ | - 3 - 4 H |
| | 4,5 | -4 - 5 | - 4 | 4 | - 4 |

## Patentansprüche

1. Verfahren zur Verbesserung der fotochemischen Stabilität von Färbungen auf Polyesterfasermaterialien mit UV-Absorbern, dadurch gekennzeichnet, dass man das Fasermaterial vor dem Färben oder während des Färbens mit einer Verbindung der Formel

(1) 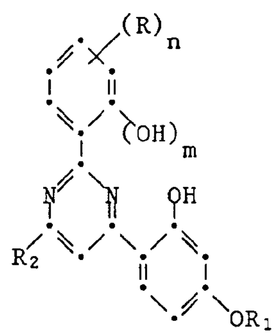

behandelt, worin
R $C_1$-$C_{18}$-Alkyl, $C_1$-$C_{18}$-Alkoxy, Halogen oder Hydroxy,
$R_1$ $C_1$-$C_{12}$-Alkyl
$R_2$ Wasserstoff, Halogen, $C_1$-$C_{18}$-Alkyl, $C_1$-$C_{12}$-Alkylamino, Hydroxy-$C_1$-$C_{12}$-alkylamino, Ar-$C_1$-$C_3$-alkylamino, Alkoxyalkylamino mit insgesamt 2-8 C-Atomen, $C_2$-$C_{18}$-Alkenyl, $C_1$-$C_{18}$-Alkoxy, durch Hydroxy, Carboxy oder $C_2$-$C_5$-Alkoxycarbonyl substituiertes $C_1$-$C_{18}$-Alkoxy, $C_2$-$C_{18}$-Alkenyloxy, Phenyl oder durch Halogen, $C_1$-$C_4$-Alkyl, Hydroxy-$C_1$-$C_4$-alkyl, $C_2$-$C_{18}$-Alkenyloxy, $C_1$-$C_{18}$Alkoxy, Hydroxy, Hydroxy-$C_1$-$C_4$-alkoxy oder Carboxy-$C_1$-$C_4$-alkoxy substituiertes Phenyl,
m 0 oder 1 und
n 0, 1 oder 2
bedeuten.

2. Verfahren gemäss Anspruch 1, dadurch gekennzeichnet, dass man eine Verbindung der Formel

(2)

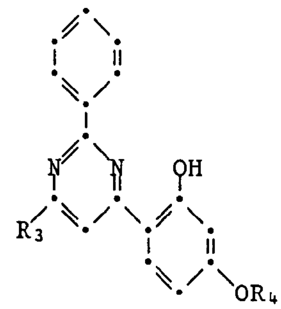

verwendet worin

$R_3$ Wasserstoff, Chlor, $C_1$-$C_4$-Alkyl, $C_1$-$C_8$-Alkylamino, Hydroxy-$C_1$-$C_4$-alkylamino, Benzylamino, Phenethylamino, $C_1$-$C_3$-Alkoxy-$C_2$-$C_4$-alkylamino, Phenyl oder durch $C_1$-$C_4$-Alkyl, Hydroxy, $C_1$-$C_{18}$-Alkoxy, $C_2$-$C_{18}$-Alkenyloxy, Hydroxy-$C_1$-$C_4$-alkoxy oder Carboxy-$C_1$-$C_4$-alkoxy substituiertes Phenyl und

$R_4$ $C_1$-$C_8$-Alkyl

bedeuten.

3. Verfahren gemäss einem der Ansprüche 1 und 2, dadurch gekennzeichnet, dass man eine Verbindung der Formel

(3)

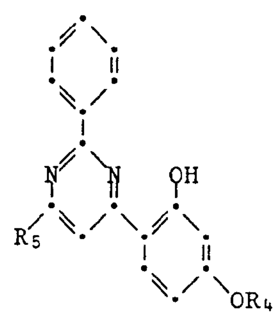

verwendet, worin

$R_4$ $C_1$-$C_8$-Alkyl und

$R_5$ $C_1$-$C_4$-Alkyl, Chlor, Phenyl, o-Hydroxyphenyl, $C_1$-$C_8$-Alkylamino, Hydroxy-$C_1$-$C_4$-alkylamino, Phenetylamino oder $C_1$-$C_3$-Alkoxy-$C_2$-$C_3$-alkylamino

bedeuten.

4. Verfahren gemäss Anspruch 1, worin $R_2$ Wasserstoff, Halogen, $C_1$-$C_{18}$-Alkyl, $C_2$-$C_{18}$-Alkenyl, $C_1$-$C_{18}$-Alkoxy, durch Hydroxy, Carboxy oder $C_2$-$C_5$-Alkoxycarbonyl substituiertes $C_1$-$C_{18}$-Alkoxy, $C_2$-$C_{18}$-Alkenyloxy, Phenyl oder durch Halogen, $C_1$-$C_4$-Alkyl, Hydroxy-$C_1$-$C_4$-alkyl, $C_2$-$C_{18}$-Alkenyloxy, $C_1$-$C_{18}$-Alkoxy, Hydroxy, Hydroxy-$C_1$-$C_4$-alkoxy oder Carboxy-$C_1$-$C_4$-alkoxy substituiertes Phenyl bedeutet.

5. Verfahren gemäss Anspruch 2, worin $R_3$ Wasserstoff, Chlor, $C_1$-$C_4$-Alkyl, Phenyl oder durch $C_1$-$C_4$-Alkyl, Hydroxy, $C_1$-$C_{18}$-Alkoxy, $C_2$-$C_{18}$-Alkenyloxy, Hydroxy-$C_1$-$C_4$-alkoxy oder Carboxy-$C_1$-$C_4$-alkoxy substituiertes Phenyl bedeutet.

6. Verfahren gemäss Anspruch 3, worin $R_5$ $C_1$-$C_4$-Alkyl, Chlor, Phenyl oder o-Hydroxyphenyl bedeutet.

7. Verfahren gemäss einem der Ansprüche 1 bis 6, dadurch gekennzeichnet, dass man die einzusetzende Verbindung der Formel (1), (2) oder (3) in einer Menge von 0,5 bis 7,5 Gew.-% des Fasermaterials verwendet.

8. Verfahren gemäss einem der Ansprüche 1 bis 7, dadurch gekennzeichnet, dass man die Verbindung der Formel (1), (2) oder (3) direkt dem Färbebad zusetzt.

9. Verbindungen der Formel

(4)

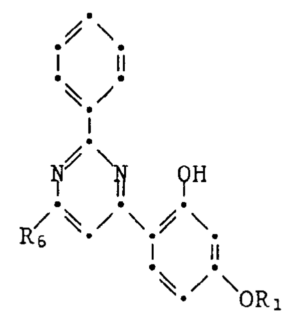

worin

$R_1$ $C_1$-$C_{12}$-Alkyl und

$R_6$ $C_1$-$C_8$-Alkylamino, Hydroxy-$C_1$-$C_4$-alkylamino, Phenethylamino oder $C_1$-$C_3$-Alkoxy-$C_2$-$C_3$-alkylamino bedeuten.

## Claims

1. A process or improving the photochemical stability of dyeings on polyester fibre materials by means of UV absorbers, which comprises treating the fibre material before or during dyeing with a compound of the formula

(1)

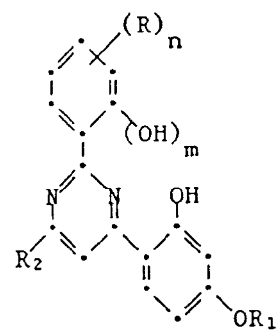

in which

R is $C_1$-$C_{18}$alkyl, $C_1$-$C_{18}$alkoxy, halogen or hydroxyl,

$R_1$ is $C_1$-$C_{12}$alkyl,

$R_2$ is hydrogen, halogen, $C_1$-$C_{18}$alkyl, $C_1$-$C_{12}$alkylamino, hydroxy-$C_1$-$C_{12}$alkylamino, aryl-$C_1$-$C_3$alkylamino, alkoxyalkylamino with a total of 2-8 carbon atoms, $C_2$-$C_{18}$alkenyl, $C_1$-$C_{18}$alkoxy, $C_1$-$C_{18}$alkoxy which is substituted by hydroxyl, carboxyl or $C_2$-$C_5$alkoxycarbonyl, $C_2$-$C_{18}$alkenyloxy, phenyl or phenyl which is substituted by halogen, $C_1$-$C_4$alkyl, hydroxy-$C_1$-$C_4$alkyl, $C_2$-$C_{18}$alkenyloxy, $C_1$-$C_{18}$alkoxy, hydroxyl, hydroxy-$C_1$-$C_4$alkoxy or carboxy-$C_1$-$C_4$alkoxy,

m is 0 or 1 and

n is 0, 1 or 2.

2. A process according to claim 1, wherein a compound of the formula

(2)

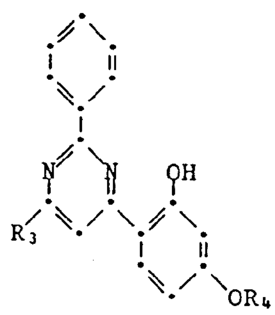

in which $R_3$ is hydrogen, chlorine, $C_1$-$C_4$alkyl, $C_1$-$C_8$alkylamino, hydroxy-$C_1$-$C_4$alkylamino, benzylamino, phenethylamino, $C_1$-$C_3$alkoxy-$C_2$-$C_4$alkylamino, phenyl or phenyl which is substituted by $C_1$-$C_4$alkyl, hydroxyl, alkoxy, $C_2$-$C_{18}$alkenyloxy, hydroxy-$C_1$-$C_4$alkoxy or carboxy-$C_1$-$C_4$alkoxy and
$R_4$ is $C_1$-$C_{18}$alkyl, is used.

3. A process according to any one of claims 1 and 2, wherein a compound of the formula

(3)

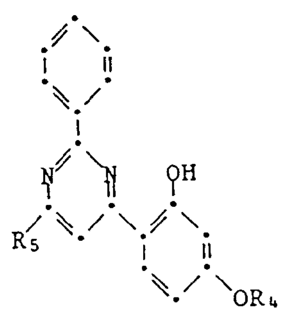

in which
$R_4$ is $C_1$-$C_8$alkyl and
$R_5$ is $C_1$-$C_4$alkyl, chlorine, phenyl, o-hydroxyphenyl, $C_1$-$C_8$alkylamino, hydroxy-$C_1$-$C_4$alkylamino, phenethylamino or $C_1$-$C_3$alkoxy-$C_2$-$C_3$alkylamino, is used.

4. A process according to claim 1, wherein $R_2$ is hydrogen, halogen, $C_1$-$C_{18}$alkyl, $C_2$-$C_{18}$alkenyl, $C_1$-$C_{18}$alkoxy, $C_1$-$C_{18}$alkoxy which is substituted by hydroxyl, carboxyl or $C_2$-$C_5$alkoxycarbonyl, $C_2$-$C_{18}$alkenyloxy, phenyl or phenyl which is substituted by halogen, $C_1$-$C_4$alkyl, hydroxy-$C_1$-$C_4$alkyl, $C_2$-$C_{18}$alkenyloxy, $C_1$-$C_{18}$alkoxy, hydroxyl, hydroxy-$C_1$-$C_4$alkoxy or carboxy-$C_1$-$C_4$alkoxy.

5. A process according to claim 2, wherein $R_3$ is hydrogen, chlorine, $C_1$-$C_4$alkyl, phenyl or phenyl which is substituted by $C_1$-$C_4$alkyl, hydroxyl, $C_1$-$C_{18}$alkoxy, $C_2$-$C_{18}$alkenyloxy, hydroxy-$C_1$-$C_4$alkoxy or carboxy-$C_1$-$C_4$alkoxy.

6. A process according to claim 3, wherein $R_5$ is $C_1$-$C_4$alkyl, chlorine, phenyl or o-hydroxyphenyl.

7. A process according to any one of claims 1 to 6, wherein the compound of the formula (1), (2) or (3) to be employed is used in an amount of 0.5 to 7.5 % by weight of the fibre material.

8. A process according to any one of claims 1 to 7, wherein the compound of the formula (1), (2) or (3) is added directly to the dyebath.

9. A compound of the formula

(4)

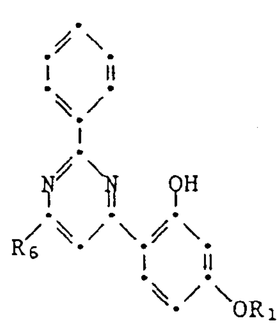

in which
$R_1$ is $C_1$-$C_{12}$alkyl and
$R_6$ is $C_1$-$C_8$alkylamino, hydroxy-$C_1$-$C_4$alkylamino, phenethylamino or $C_1$-$C_3$alkoxy-$C_2$-$C_3$alkylamino.

## Revendications

1. Procédé pour améliorer la stabilité photochimique de teintures sur des matériaux fibreux en polyester à l'aide d'absorbeurs d'UV, caractérisé en ce qu'on traite le matériau fibreux, avant la teinture ou pendant la teinture, avec un composé de formule

(1)

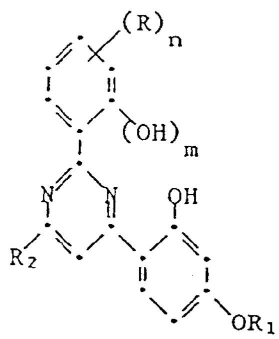

où
R représente un groupe alkyle en $C_1$-$C_{18}$, un groupe alcoxy en $C_1$-$C_{18}$, un atome d'halogène ou un groupe hydroxy,
$R_1$ représente un groupe alkyle en $C_1$-$C_{12}$,
$R_2$ représente un atome d'hydrogène, un atome d'halogène, un groupe alkyle en $C_1$-$C_{18}$, alkylamino en $C_1$-$C_{12}$, hydroxyalkylamino en $C_1$-$C_{12}$, arylalkyl(en $C_1$-$C_3$)amino, un groupe alcoxyalkylamino avec, en tout, 2 à 8 atomes de carbone, un groupe alcényle en $C_2$-$C_{18}$, alcoxy en $C_1$-$C_{18}$, alcoxy en $C_1$-$C_{18}$ substitué par un groupe hydroxy, carboxy ou alcoxycarbonyle en $C_2$-$C_5$, un groupe alcényloxy en $C_2$-$C_{18}$, phényle ou phényle substitué par un atome d'halogène, un groupe alkyle en $C_1$-$C_4$, hydroxyalkyle en $C_1$-$C_4$, alcényloxy en $C_2$-$C_{18}$, alcoxy en $C_1$-$C_{18}$, hydroxy, hydroxyalcoxy en $C_1$-$C_4$ ou carboxy-(alcoxy en $C_1$-$C_4$),
m signifie 0 ou 1 et
n signifie 0, 1 ou 2.

2. Procédé selon la revendication 1, caractérisé en ce qu'on utilise un composé de formule

(2)

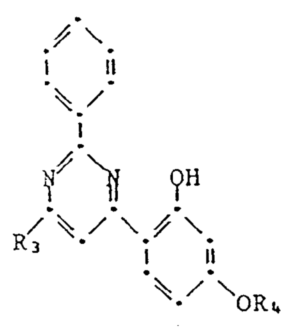

où
$R_3$ représente un atome d'hydrogène, un atome de chlore, un groupe alkyle en $C_1$-$C_4$, alkylamino en $C_1$-$C_8$, hydroxyalkylamino en $C_1$-$C_4$, benzylamino, phénéthylamino, (alcoxy en $C_1$-$C_3$)(alkyl en $C_2$-$C_4$)amino, phényle ou phényle substitué par un groupe alkyle en $C_1$-$C_4$, hydroxy, alcoxy en $C_1$-$C_{18}$, alcényloxy en $C_2$-$C_{18}$, hydroxyalcoxy en $C_1$-$C_4$ ou carboxy-(alcoxy en $C_1$-$C_4$), et
$R_4$ représente un groupe alkyle en $C_1$-$C_8$.

3. Procédé selon l'une des revendications 1 et 2, caractérisé en ce qu'on utilise un composé de formule

(3)

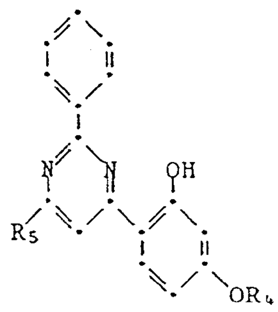

où

$R_4$ représente un groupe alkyle en $C_1$-$C_8$, et

$R_5$ représente un groupe alkyle en $C_1$-$C_4$, un atome de chlore, un groupe phényle, o-hydroxyphényle, alkylamino en $C_1$-$C_8$, hydroxyalkylamino en $C_1$-$C_4$, phénéthylamino ou (alcoxy en $C_1$-$C_3$)(alkyl en $C_2$-$C_3$)-amino.

4. Procédé selon la revendication 1, où $R_2$ représente un atome d'hydrogène, un atome d'halogène, un groupe alkyle en $C_1$-$C_{18}$, alcényle en $C_2$-$C_{18}$, alcoxy en $C_1$-$C_{18}$, alcoxy en $C_1$-$C_{18}$ substitué par un groupe hydroxy, carboxy ou alcoxycarbonyle en $C_2$-$C_5$, un groupe alkényloxy en $C_2$-$C_{18}$, phényle ou phényle substitué par un atome d'halogène, un groupe alkyle en $C_1$-$C_4$, hydroxyalkyle en $C_1$-$C_4$, alcényloxy en $C_2$-$C_{18}$, alcoxy en $C_1$-$C_{18}$, hydroxy, hydroxyalcoxy en $C_1$-$C_4$ ou carboxy-(alcoxy en $C_1$-$C_4$).

5. Procédé selon la revendication 2, où $R_3$ représente un atome d'hydrogène, un atome de chlore, un groupe alkyle en $C_1$-$C_4$, phényle ou phényle substitué par un groupe alkyle en $C_1$-$C_4$, hydroxy, alcoxy en $C_1$-$C_{18}$, alcényloxy en $C_2$-$C_{18}$, hydroxyalcoxy en $C_1$-$C_4$ ou carboxy-(alcoxy en $C_1$-$C_4$).

6. Procédé selon la revendication 3, où $R_5$ représente un groupe alkyle en $C_1$-$C_4$, un atome de chlore, un groupe phényle ou o-hydroxyphényle.

7. Procédé selon l'une des revendications 1 à 6, caractérisé en ce qu'on utilise le composé de formule (1), (2) ou (3) à incorporer, en une quantité de 0,5 à 7,5% en poids par rapport au matériau fibreux.

8. Procédé selon l'une des revendications 1 à 7, caractérisé en ce qu'on ajoute le composé de formule (1), (2) ou (3) directement au bain de teinture.

9. Composés de formule

(4)

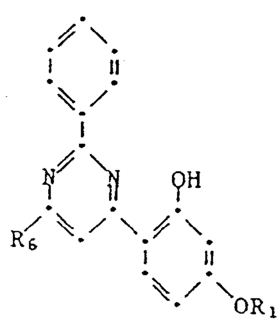

où

$R_1$ représente un groupe alkyle en $C_1$-$C_{12}$, et

$R_5$ représente un groupe alkylamino en $C_1$-$C_8$, hydroxyalkylamino en $C_1$-$C_4$, phénéthylamino ou (alcoxy en $C_1$-$C_3$)(alkyl en $C_2$-$C_3$)amino.